# EUROPEAN PATENT APPLICATION

(11) **EP 3 189 860 A1**
(43) Date of publication of application: **12.07.2017**
(21) Application number: 17150516.7
(22) Date of filing: 06.01.2017
(51) Int. Cl.: A61M 1/00, A61M 25/01

(54) **MOTORIZED CHEST DRAINAGE SYSTEM**

(30) Priority: 07.01.2016 US 201662275829 P; 14.12.2016 US 201615378532
(71) Applicant: Covidien LP, Mansfield, MA 02048 (US)
(72) Inventor: SHAH, Sachin, Milford, CT 06460 (US); RACENET, David, Killingworth, CT 06419 (US); MUKHERJEE, Nilay, Burlington, MA 01803 (US)
(74) Representative: Maschio, Antonio

(57) **Abstract**

A chest drainage system is disclosed, including a flexible tube with an articulable tip and a control assembly. The control assembly is operated by a motor and includes an actuation assembly. The actuation assembly is operatively coupled with the flexible tube and is adapted to articulate the tip.

## Description

### CROSS-REFERENCE TO RELATED APPLICATIONS

This application claims the benefit of and priority to U.S. Provisional Patent Application No. 62/275,829 filed January 7, 2016, the entire disclosure of which is incorporated by reference herein.

### TECHNICAL FIELD

The present disclosure relates generally to a medical drainage system, and in particular, a motorized chest drainage system and a method of use thereof.

### BACKGROUND

The human pleura and chest wall are normally in opposition. A variety of iatrogenic or organic states can least to interposition of either gas or fluid between the pleura and chest wall, which if unchecked may compromise the lungs and may cause a variety of sequelae, from shortness of breath to profound hypoxia/hypotension. Thoracic surgical procedures where the pleura are violated may naturally result in pneumothorax; also, occasionally people may spontaneously pop diseased lung tissue, which also results in pneumothorax. In lung resection procedures, this problem can persist, therefore chest drainage systems are routinely placed within a patient's pleural space to treat this pneumothorax and minimize the accumulation of fluid. In certain disease states including trauma, pneumonia, and cancer, fluid may accumulate in the pleural space more quickly than it can be evacuated. As noted, this may lead to unchecked compression of the lungs leading to the aforementioned sequelae. Frequently these fluid collections move. A chest drainage system with a motorized tip may assist in the removal of fluid within a patient's pleural cavity.

A basic chest drainage system includes a chest tube and drainage canister. Advancements have been made to the basic system. A chest drainage system can now include a suction system, de-clogging system, or a sensor system or any combination thereof. The inclusion of these systems permits a chest drainage system to appropriately handle the dynamic atmosphere within the pleural cavity. However, despite these advancements, chest drainage systems may still lack mobility once placed within a patient's pleural cavity.

Accordingly, a motorized chest drainage system capable of being repositioned after being placed within the patient's pleural cavity is desirable.

### SUMMARY

The present disclosure is directed to a motorized chest drainage system. The motorized chest drainage system includes a flexible tube having proximal and distal ends, a tip positioned at the distal end, an articulation assembly operatively coupled with the flexible tube, and a control assembly which is operated by a motor. The control assembly is operatively coupled with the articulation assembly, and the articulation assembly is adapted to articulate the tip.

In one embodiment, the chest drainage system may include a suction source. This suction source may be coupled with a sensor unit that will collect data on the suction pressure, fluid flow rate, or content type or any combination thereof. The sensor may also be coupled with a data processor, which will evaluate the data collected by the sensor. The data evaluated by the data processor may be communicated to a display located on the case of the device. The case may house the articulation assembly, the motorized control assembly, the suction source, sensor, and data processor. The case may also include controls for the articulation assembly, a motor control, a power outlet or battery, or both, and a fluid reservoir that will collect all fluids being drained from a patient's pleural cavity.

In another embodiment, there may be an optionally detachable sensor unit that will attach in-line with the chest drainage tube, between the distal end of the chest tube and the case of the drainage system. The detachable sensor unit will connect to the chest tube via a set of fittings at each end of the detachable sensor unit. The detachable sensor unit might contain one or more sensors that could monitor various parameters of the fluids and/or solids that travel through the detachable sensor unit. These parameters include, but are not limited to, pressure, flow rate, pH, presence of blood, carbon dioxide levels, glucose levels, as well as other parameters of the contents of the chest tube. The detachable sensor unit may also contain wireless communications capabilities. The detachable sensor unit may also contain a data processor, which would analyze the data collected by the various sensors in the detachable sensor unit, and wirelessly communicate information about the analyzed data to mobile communication devices, such as mobile phones or pagers carried by monitoring personnel. Additionally, the detachable sensor unit may have a display system, which can display data collected by the various sensors. The detachable sensor unit may also contain a replaceable battery to provide power for the sensors, data processor, display system, and wireless communications units.

In another embodiment, the articulation assembly may be remotely operated, programmed to have a set oscillation pattern, programmed to have a user defined pattern or any combination thereof.

These and other features of the current disclosure will be explained in greater detail in the following detailed descriptions of the various embodiments.

### BRIEF DESCRIPTION OF THE DRAWINGS

Various embodiments of the present disclosure are described herein below with reference to the drawings, wherein:
FIG. **1** is a perspective view of a motorized chest drainage system in accordance with the present disclosure;
FIG. **2** is an interior view of a motorized chest drainage system as shown in FIG. **1**;
FIG. **3** is a rear perspective view of a control assembly, including multiple connecting members;
FIG. **4** is an enlarged area of detail view of the first and second segments as shown in FIG. **1**, showing bi-directional articulation of the first and second segments in a first plane and a second plane;
FIG. **5** is a perspective view of the control assembly shown in FIG. **3** positioned within an outer housing and coupled to a motor;
FIG. **6** is a basic schematic design for the display system; and
FIG. **7** is a perspective view of a detachable sensor unit;
FIG. **8** is an interior view of the detachable sensor unit as shown in FIG. 7; and
FIG. **9** is a basic schematic design for the display system within the sensor unit.

Other features of the present disclosure will become apparent from the following detailed description taken in conjunction with the accompanying drawings, which illustrate, by way of example, the principles of the present disclosure.

### DETAILED DESCRIPTION

Embodiments of the presently disclosed motorized chest drainage system are described in detail with reference to the drawings, in which like reference numerals designate identical or corresponding elements in each of the several views. As used herein, the term "clinician" refers to a doctor, a nurse, or any other care provider. Throughout this description, the term "distal" refers to that portion of the tool or component thereof which is farther from the user while the term "proximal" refers to the portion of the tool or component thereof which is closer to the user. The presently disclosed chest drainage system is usable in openings through a patient's tissue.

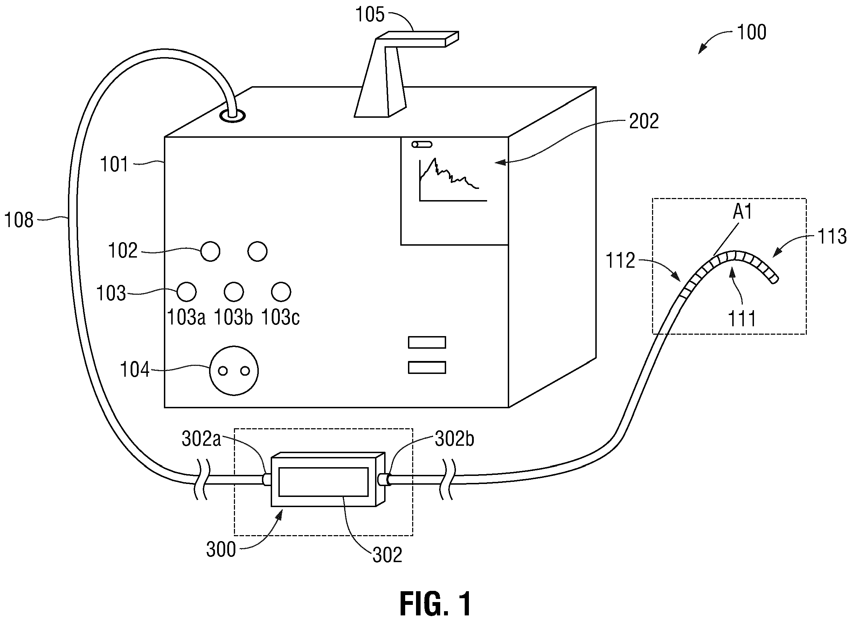

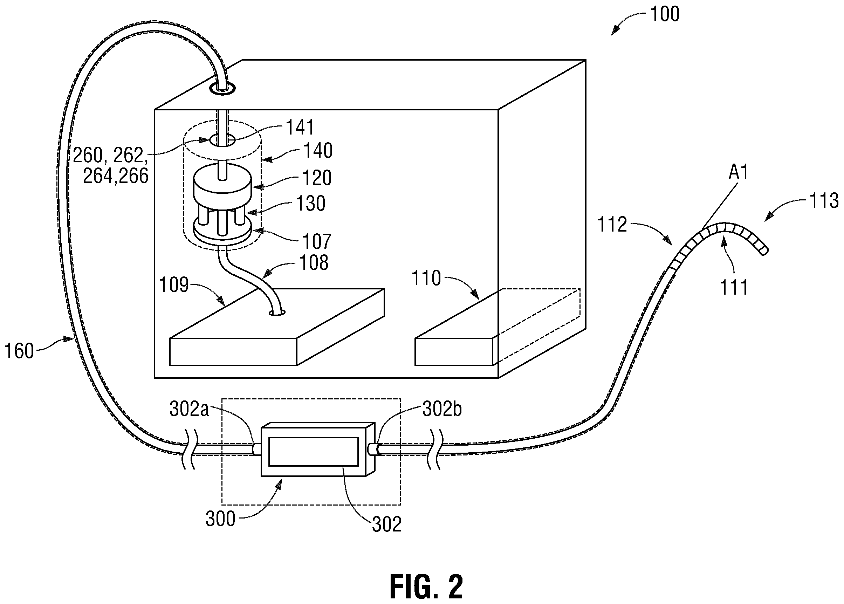

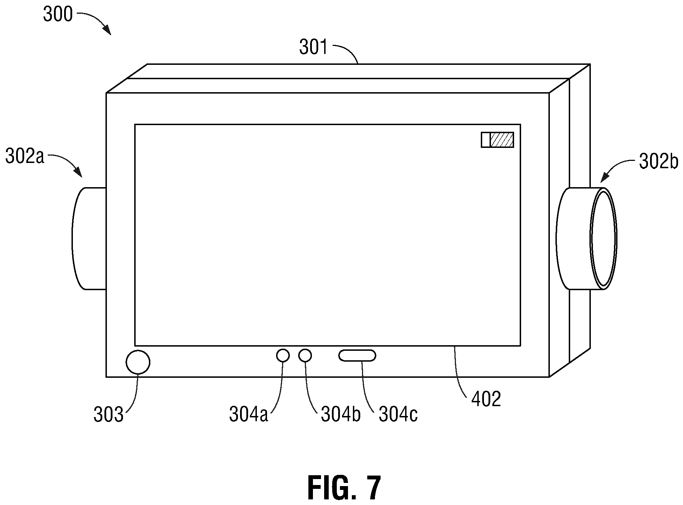

Referring initially to FIG. **1**, a motorized chest drainage system **100** is shown. Motorized chest drainage system **100** includes a case **101**, which houses select components of the chest drainage system **100**. The case **101** includes actuation controls **102** that permit a user to operate an actuation assembly **130** (FIG. **2**), motor controls **103** that allow the user to choose between remotely controlling the actuation assembly **130** or a set oscillation pattern or a user defined pattern, and a display **202**, all of which will be described in further detail below. Also, the case **101** includes an outlet **104** and a handle **105**. Extending from the case **101** is a chest tube **108** having an articulable tip **111**. The motorized chest drainage system **100** may include an optional, detachable sensor unit **300**. Sensor unit **300** is shown connected in-line with the chest tube **108**. With additional reference to FIG. 7, the sensor unit 300 includes a set of fittings **302a** and **302b** at each end to allow attachment to the chest drainage tube 108. The fittings **302a** and **302b** may be made of a suitable biocompatible material such as plastic or metal. The fittings **302a** and **302b** may be adhered, welded, snap-fit, press fit, or otherwise coupled to the sensor **300** unit. These fittings **302a** and **302b** allow chest tube **108** to be removably coupled to sensor **300**.

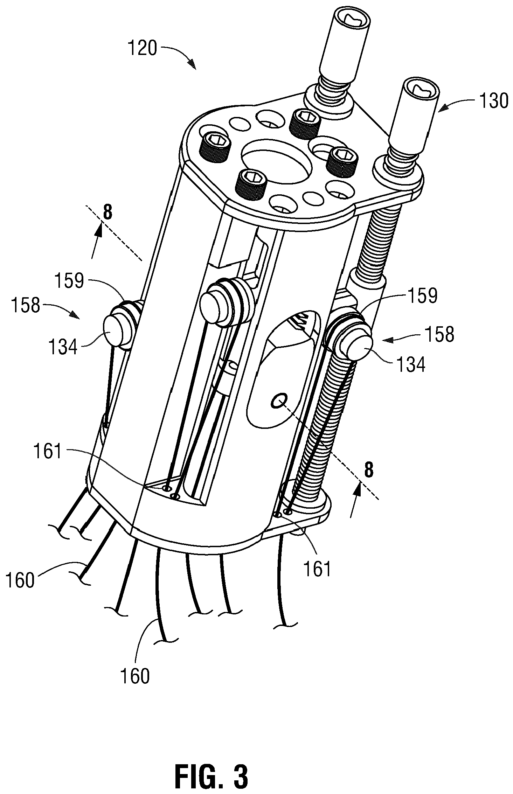

Referring now to FIG. **2**, the motorized chest drainage system **100** is shown in further detail. The front plate of the case **101** is removed to show a fluid reservoir **109**, a battery **110**, and a housing **140** (shown in phantom view). The housing **140** contains a control assembly **120**, an articulation assembly **130**, sensors **260**, **262**, **264**, and **266** and a motor **107**. Housing **140** may have any suitable shape to accommodate control assembly **120**, articulation assembly **130**, sensors **260**, **262**, **264**, and **266** and motor **107** and includes an aperture to receive one end of the chest tube **108**. Also, housing **140** includes connecting apertures 141 to receive connecting members **160** (FIG. **3**). The fluid reservoir **109** is removable so that collected fluids may be properly disposed of. Alternatively, fluid reservoir **109** may be attachable to a drain system such that any collected fluids are transferred to the drain system.

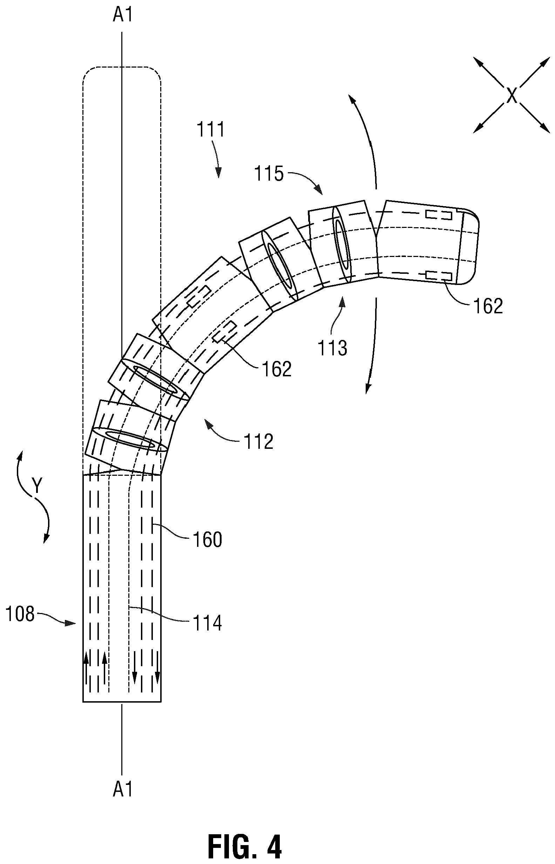

As seen in FIG. **3**, control assembly **120** includes two actuation assemblies **130**. Actuation assemblies **130** and associated components are substantially similar to each other, and cooperate to effect actuation of the articulable tip **111**. More than two actuation assemblies **130** or less than two actuation assemblies **130** may be present in control assembly **120** to suit the particular needs of articulation. Each actuation assembly **130** is shown fully assembled, with connecting members **160** attached to portions of the actuation assembly **130**. Connecting members **160** couple the actuation assemblies **130** to portions of the articulable tip **111** (FIG. **2**). Connecting members **160** are shown disposed over a portion of pulleys **158** within track **159**. Pulleys **158** are retained by securing members **134**. Thus, when pulleys **158** rotate about securing members **134**, they displace a portion of connecting members **160** disposed in the track **159**. While connecting members **160** are shown as cables, connecting members **160** may be wires or other tensile elements, or may be rigid elements such as bars or links. Connecting members **160** include a proximal end **161** and a distal end **162** (FIG. **4**). Ends **161, 162** of the connecting members **160** may be defined by a ferrule, or may be knotted or otherwise defined.

Referring now to FIG. **4**, articulable tip **111** includes at least a first segment **112** and a second segment **113**. Second segment **113** is disposed distally of the first segment **112**. The first segment **112** and the second segment **113** are capable of independent movement relative to the longitudinal axis **A1** and to each other. Connecting members **160** couple the actuation assemblies **130** to portions of the articulable tip **111**.

As forces are transmitted to the connecting members **160** (shown in phantom view), displacement of the first and second segments **112**, **113** is effected in a first plane, i.e., plane X (across the page) and a second plane, i.e., plane Y (into and out of the page). Connecting members **160** associated with a first actuation assembly **130** may be attached to opposing surfaces in each of the first and second segments **112**, **113** to effect articulation in plane X, and connecting members **160** associated with a second articulation assembly **130** may be attached to opposing surfaces in each of the first and second segments **112**, **113** and radially spaced from the connecting members **160** of the first articulation assembly **130** to effect articulation in plane Y. Forward and reverse engagement of the pair of actuation assemblies **130** allows for bi-directional articulation of the first and second segments **112**, **113** in both plane X and plane Y. Accordingly, articulable tip **111** can be articulated in opposing directions in multiple planes. The first and second segments **112**, **113** of the articulable tip **111** may be continuous flexible members, or may include independently movable members **115** that, when assembled, engage in a manner such that each movable member **115** is free to pivot relative to an adjacent movable member **115**.

Chest tube **108** includes a lumen **114** (shown in phantom). Lumen **114** may be a separate tube having flexibility or flexible portions to correspond to the first and second segments **112**, **113** of the articulable tip **111**. The lumen **114** provides an access pathway between a distal end of the chest tube **108** and a proximal portion thereof (e.g., the distal tip and the reservoir). The lumen **114** may be used for irrigation, vacuum, suction, de-clogging, or providing instrument access into the patient's pleural cavity. Suction may be delivered by a number of different methods. In one embodiment, suction may be provided by a combined vacuum/pressure system, which will be connected to lumen **114** and chest tube **108**. The combined vacuum/pressure system will provide negative pressure to lumen **114**, which will allow the fluids contained within the patient's pleural cavity to be drawn out quicker. The user may reverse the pressure within the lumen **114** to de-clog chest tube **108** using positive pressure to dislodge an obstruction. In another embodiment, de-clogging can be achieved by temporarily removing chest tube **108** from the pleural cavity and manually removing the obstruction. In another embodiment, de-clogging might be achieved by a morcellator disposed inside lumen **114** that grinds up any blocking material into small enough pieces that will adequately pass through the drain and out of the patient's pleural cavity. De-clogging may also be achieved by means of an obturator to expel the blockage. In another embodiment, de-clogging might be made unnecessary by placing a filter at the distal end of the tube to prevent anything other than liquid or smaller material to pass through. Also, in another embodiment the chest tube **108** may include two or more lumens (not shown).

A detailed description of articulation assembly and methods of effecting articulation of the articulable portion are found, for example, in U.S. Patent Application Publication No. 2012-0310220, the entire contents of which is incorporated herein by reference.

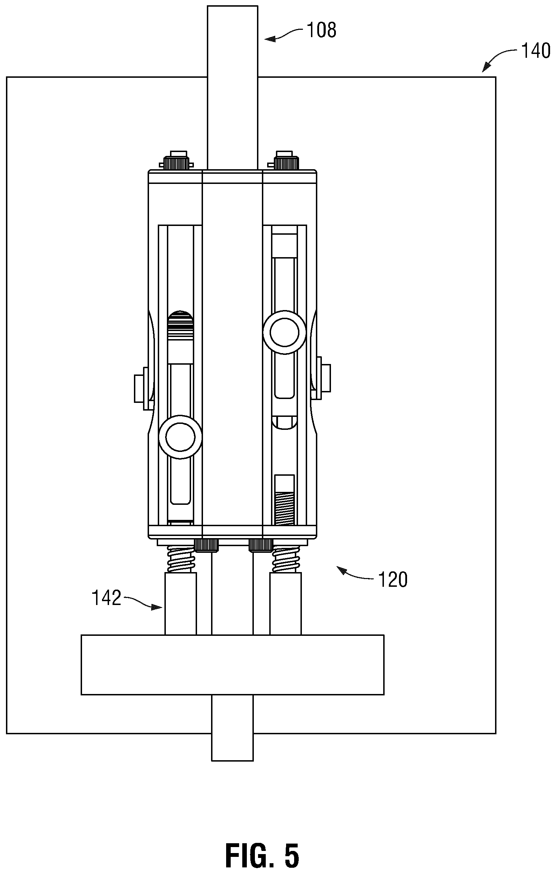

Referring to FIGS. **2** and **5****,** control assembly **120** is shown as placed vertically within the housing **140**. The control assembly **120** may also be placed horizontally, angled or any desired position. Control assembly **120** is connected to the motor **107**. The connection between the control assembly **120** and motor **107** can be achieved by a number of different connections. In one embodiment, the control assembly **120** includes drive members **141** which may have surface features to engage gear couplers (not shown) within motor **107**. In another embodiment, the drive members **141** are connected to the shaft of the motor **107** via shaft couplers (not shown). The shaft couplers may be adhered, welded, snap-fit, press fit, or otherwise coupled to the drive members **141** and the motor **107**. The motor **107** provides the mechanical energy necessary for the actuation assemblies **130** to transmit force to move connecting members **160** and thus articulate the articulable tip **111**. Additionally, motor **107** includes an aperture to allow chest tube **108** to pass through and connect to the fluid reservoir **109** located beneath the housing **140**. In another embodiment, motor **107** may be offset to avoid interference with the chest tube **108**.

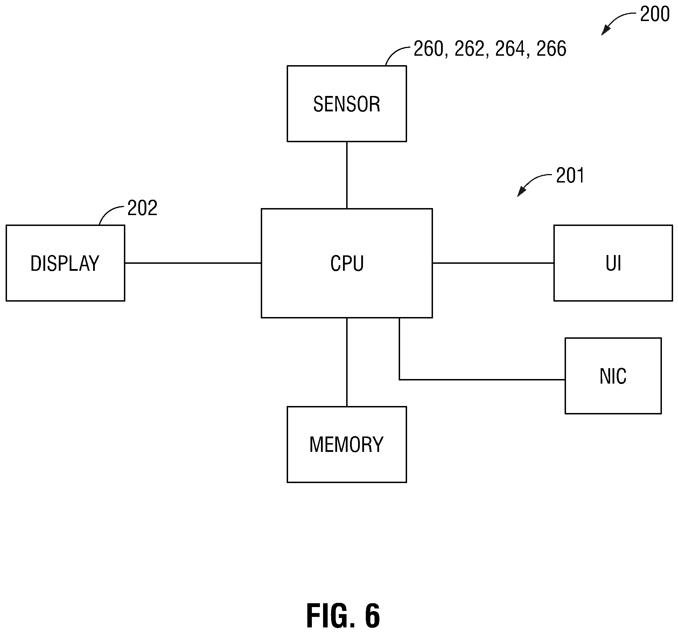

Referring additionally to FIG. **1**, the actuation assemblies **130** can be remotely controlled. The user remotely controls the actuation assemblies **130** by actuation controls **102** on case **101**. The actuation controls **102** will trigger motor **107** to transmit the desired force to move actuation assemblies **130**, which will pull the connecting members **160** and thus articulate the articulable tip **111** in the X plane and/or Y plane (FIG. **4**). The user may remotely control the actuation assemblies **130** to correct any migration of the articulable tip **111**, or can articulate the articulable tip **111** between various locations to treat all pleural effusions. This will ensure the articulable tip **111** remains in the correct location within the pleural cavity at all times. Also, actuation assemblies **130** and motor **107** may be programmed to have a predefined oscillation pattern or a user defined pattern. These options may be achieved in a number of different methods. In one embodiment, the data processor (FIG. 6) may include a memory which can store an algorithm to control a set oscillation pattern, an algorithm to control a user defined pattern, a predefined program or any combination thereof. The data processor will then execute the program or algorithm stored in the memory. Both the set oscillation pattern and user defined pattern may correlate with the volume of fluid being suctioned from the patent's pleural cavity, the suction pressure, fluid flow rate, or volume of fluid contained with fluid reservoir or any combination thereof. Motor controls **103** allow the user to select between the remote control option, the set oscillation option, and the user defined option. Motor controls **103** include a plurality of user actuated buttons, knobs, or switches. A first switch **103a** allows the user to select the remote control option. A second switch **103b** allows the user to select the oscillation option. A third switch **103c** allows the user to select the user defined option. Motor controls **103** allow the user to enter user defined pattern. In one embodiment, switches **103a**, **103b** and **103c** will be configured so that once the desired switch is selected by the user the unselected switches will be disabled.

Turning to FIG. **6**, display system **200** includes a data processor **201** and display **202**. The data processor **201** may include a central processing unit, user interface, memory, sensor, and wireless component. A basic schematic of the display system **200** is shown in FIG. **6**. As it is used in this description, "user interface" generally refers to any visual, graphical, tactile, audible, sensory, or other mechanism for providing information to and/or receiving information from a user or other entity. The term "user interface" as used herein may refer to an interface between a human user (or operator) and one or more devices to enable communication between the user and the device(s). Example of user interface that may be employed in various embodiments of the present disclosure include, without limitation, switches, potentiometers, buttons, dials, sliders, a mouse, a pointing device, a keyboard, a keypad, joysticks, trackballs, display screen, various types of graphical user interfaces (GUIs), touch screens, microphones and other types of sensors or devices that may receive some form of human-generated stimulus and generate a signal in response thereto. As it is used herein, "computer" generally refers to anything that transforms information in a purposeful way.

The display system **200** described herein may also utilize one or more controllers to receive various information and transform the received information to generate an output. The controller may include any type of computing device, computational circuitry, or any type of processor or processing circuitry capable of executing a series of instructions that are stored in a memory. The controller may include multiple processor and/or multicore central processing units (CPUs) and may include any type of processor, such as a microprocessor, digital signal processor, microcontroller, or the like. The controller may also include a memory to store data and/or algorithms to perform a series of instructions.

A network interface card (NIC) or other suitable network interface utilizes any known communication methods for transmitting and/or receiving data to or from sensors **260**, **262**, **264**, and **266**.

Display screen **202** (FIG. **1**) may include a liquid crystal display, a light emitting diode display or the like.

Sensor **260** may be a pressure sensor for monitoring pleural pressure. Pressure sensors generate a signal related to the pressure being measured. Pressure sensors can be classified in terms of pressure ranges they measure, temperature ranges of operation, and most importantly the type of pressure they measure. In terms of pressure type, pressure sensors can be divided into five categories. Absolute pressure sensors which measure the pressure relative to perfect vacuum pressure (0 PSI or no pressure). Gauge pressure sensors may be used in different applications because it can be calibrated to measure the pressure relative to a given atmospheric pressure at a given location. Vacuum pressure sensors are used to measure pressure less than the atmospheric pressure at a given location. Sealed pressure sensors are similar to the gauge pressure sensors except that it is previously calibrated by its manufacturer to measure pressure relative to a sea level pressure. Sensor **260** may be configured to monitor pleural pressure within a predetermined range, and will trigger an alert to a clinician if the pleural pressure is outside that predetermined range. The predetermined range of sensor **260** may range between -4 and -20 cmH₂O with a sensitivity of 1 cmH₂O; however, the predetermined range of sensor **260** is not limited to this specified range, and may be greater or less. Also, a visual signal, audio signal or both may alert a clinician when the pleural pressure reaches a predetermined level, for example a clinician may be alerted when the pleural pressure equals 0 cm*H*₂*O* and/or is outside of the predetermined range by ± 5 cm*H*₂*O*. In another embodiment sensor **260** may monitor fluid pressure. Additionally, a single pressure sensor can be used to measure fluid level in a container.

Sensor **260** may also be a sensor for monitoring fluid (e.g., liquid or gas) flow rate. Flow rate sensors generate a signal related to the velocity of the measured fluid. Differential pressure sensors measure the difference between two or more pressures introduced as inputs to the sensing unit. Differential pressure sensors may also be used to measure flow or level in pressurized vessels. Sensor **260** may be configured to monitor fluid flow rate within a predetermined range, and will trigger an alert to a clinician if the fluid flow rate is outside that predetermined range. The predetermined range for sensor **260** may range between 0mL/h and 100mL/h with a sensitivity of 10 mL; however, the predetermined range of fluid flow rate is not limited to this specified range, and may be greater or less. Also, a visual signal, audio signal or both may alert a clinician when the fluid low rate reaches a predetermined rate, for example a clinician may be alerted when the fluid flow rate equals 0mL/h and/or when the fluid flow rate is outside of the predetermined range by ± 100 mL/H.

In another embodiment, the fluid flow rate may be monitored by altering the shape the fluid reservoir **109** to a tipping bucket configuration. The tipping bucket configuration may include a central pivoting cone shaped fluid reservoir with a drainage valve that is mounted on a support device, a set of calibration screws, a magnet and a magnetic sensor. The set of calibration screws are mounted on the base of the support device and beneath the fluid reservoir, with each calibration screw positioned at one end of the fluid reservoir opposite of one another. The magnetic sensor may be located at the top of the support device and magnet may be placed at the top of the fluid reservoir adjacent to the magnetic sensor. The magnetic sensor may be a number of different types of sensors, for example the magnetic sensor may be a reed switch sensor. The cone shaped fluid reservoir is configured to the support device by a centrally located pivot at the base of the fluid reservoir. The fluid reservoir is dimension to contain a predetermined volume of fluid. After the fluid reservoir is filled with the predetermined volume, the fluid reservoir pivots about the central pivot, which allows the fluid reservoir to tip to one side draining all the fluid from the drainage valve of the fluid reservoir. When the fluid reservoir tips to one side it rests on one of the set of calibration screws. The set of calibration screws are placed under the fluid reservoir to provide stability for the fluid reservoir when in the tipped position. Also, when the fluid reservoir tips to one side the magnet moves from its original central location and passes by the magnetic sensor. The volume of fluid is tracked by the number of times the magnet passes by the magnetic sensor.

The clinician may consider the information gathered by the sensors **260** in evaluating when chest tube **108** should be removed from the patient. Also, the information gathered by the sensor **260** may indicate to a clinician that the chest tube **108** is dislodged or clogged. Further, the information gathered by the sensor **260** may also indicate to a clinician that there is excess drainage or a number of other clinical indications. In another embodiment, there will be a feedback loop between the suction source and the sensor **260**. The motorized chest drainage system **100** will adjust the suction pressure based on the flow rate so as to minimize trauma to the healing tissue.

Sensor **262** may detect the pH levels of the fluid. Sensor **262** may be configured to monitor the pH levels of the fluid within a predetermined range, and will alert a clinician if the pH level is outside that predetermined range. The predetermined range for the pH level may range from 7.25 to 7.75 with a sensitivity of 0.1 pH; however, the predetermine range of the pH level is not limited to this specified range, and may be greater or less than this specified range. Also, a visual signal, audio signal or both may trigger an alert to a clinician when the pH level reaches a predetermined level within the range, for example a clinician may be alerted when the pH level is ≤ 7.5. A clinician may use the information gathered from sensor **262** about the pH level to determine the onset of an infection.

Sensor **264** may detect level of carbon dioxide (CO₂). Sensor **264** may be configured to monitor the level of CO₂ within a predetermined range, and will trigger an alert to a clinician if the level of CO₂ is outside that predetermined range. The predetermined range for the CO₂ level may range from 0.3 mmHg to 40 mmHg with a sensitivity of 0.1 mmHg; however, the predetermine range of the CO₂ level is not limited to this specified range, and may be greater or less than this specified range. Also, a visual signal, audio signal or both may trigger an alert to a clinician when the CO₂ level reaches a predetermined level, for example when the CO₂ level is > 0 mmHg and/or >8 mmHg. A clinician may use the information gathered from sensor **264** about the CO₂ level to determine if there is any air leaking from the lung.

Sensor **266** may detect the presence of blood in the fluid within chest tube 108. Sensor **266** may be configured to monitor for the presence of blood within a predetermined range, and will trigger an alert to a clinician if the amount of blood is outside that predetermined range. The predetermined range for the amount of blood may range from 0 red blood cells ("RBC") per mm³ to 100,000 RBC per mm³; however, the predetermine range of the amount of blood is not limited to this specified range, and may be greater or less than this specified range. Also, a visual signal, audio signal or both may alert a clinician when the amount of blood reaches a predetermined amount, for example when the amount of blood is > 100,000 RBC per mm³. In some embodiments, the sensor **266** will be a RGB sensor. An RGB sensor may measure the red, green, and blue components of light with the sensitivity similar to human vision. Detection of those colors will allow the sensor **266** to gather information on whether or not blood is present within the fluids within the chest tube **108**. Sensor **266** may also monitor the fluid turbidity. The color and turbidity sensed by sensor **266** may indicate a number of different clinical indications, such as:

| **Physical Appearance** | Light yellow and clear | Reddish | Cloudy, thick | "Milky" effusion - off white/ yellow |
|---|---|---|---|---|
| **Clinical Indication** | Normal appearance | Presence of blood | Presence of microorganisms and/or white blood cells | Chylothorax |

Also, a clinician may use the information gathered by sensor **266** to determine if there is a hemorrhage or the onset of an infection.

The data collected from the sensors **260**, **262**, **264**, and **266** may be considered by a clinician during treatment and may be used in determining when a patient is ready to be discharged. An algorithm can be developed to analyze data to determine if a patient may safely be discharged, thereby customizing the solution to the patient and potentially reducing the cost of a patient's healthcare.

In another embodiment, display system **200** may include addition sensors to gather information about a number of different clinical metrics. In one embodiment, the display system **200** may include a sensor that can detect glucose within the fluids draining from the patient. That sensor may be configured to monitor the presence of glucose within a predetermined range, and will trigger an alert to a clinician if the amount of glucose is outside that predetermined range. The predetermined range for the amount of glucose may range from 40 mg/dL to 125 mg/dL with a sensitivity of 10 mg/dL; however, the predetermine range of the amount of glucose is not limited to this specified range, and may be greater or less than this specified range. Also, a visual signal, audio signal or both may alert a clinician when the amount of glucose reaches a predetermined amount, for example a clinician may be alerted when the amount of glucose is ≤ 70mg/dL. A clinician may use the information gathered about the presence of glucose to determine the onset of an infection. In another embodiment, the display system **200** may include a sensor that can detect the presence of blood hematocrit. That sensor may be configured to monitor the presence of blood hematocrit within a predetermined range, and will trigger an alert to a clinician if the amount of blood hematocrit is outside that predetermined range. The predetermined range for the amount of blood hematocrit may range from 0% to 50% of blood hematocrit with a sensitivity of 5%; however, the predetermine range of the amount of blood hematocrit is not limited to this specified range, and may be greater or less than this specified range. Also, a visual signal, audio signal or both may alert a clinician when the amount of blood hematocrit reaches a predetermined amount, for example a clinician may be alerted when the amount of blood hematocrit is < 5% and/or < 25%. A clinician may use the information gathered about the amount of blood hematocrit to determine if there is hemorrhaging. In another embodiment, display system **200** may include a sensor that can detect the presence of neutrophils. The information gathered by this sensor may allow a clinician to determine the onset of an infection.

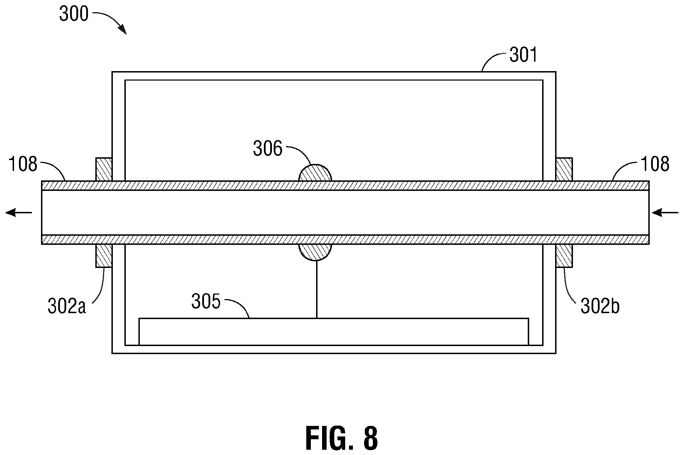

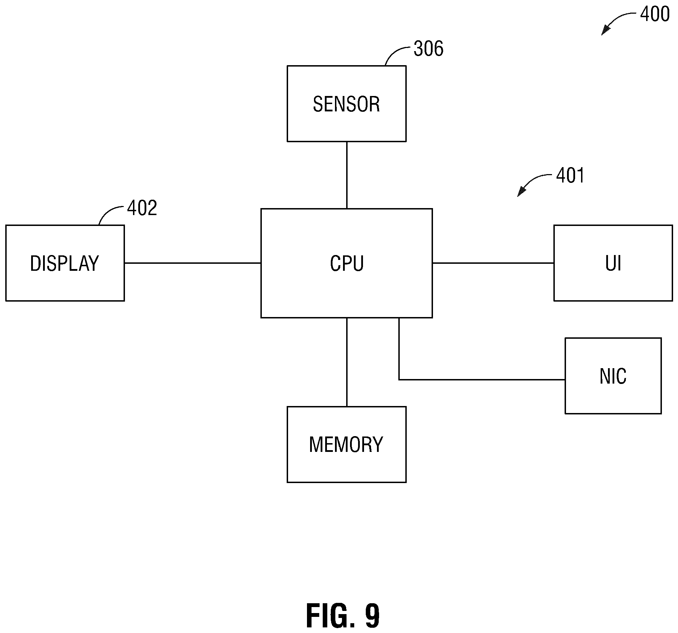

Referring now to FIGS. **7** and **8**, the sensor unit **300** is shown in further detail. Senor unit **300** is shown in FIG. **7** to include a sensor case **301**. In this embodiment sensor case **301** is manufactured into two individual pieces that may be adhered, welded, snap-fit, press fit, or otherwise coupled together. Also, sensor unit **300** includes a display system **400** (FIG. **9**), data processor **401** (FIG. **9**), display screen **402**, a power button **303**, two navigational buttons **304a** and **304b**, a selection button **304c**, a battery **305** (FIG. **8**) and a senor **306** (FIG. **8**). Display system **400** provides the clinician a local display of the information being gathered by sensors **260**, **262**, **264**, and **266** and/or a local display of the information being gathered by sensor **306** (FIG. **8**). Power button **303** allows the clinician to turn sensor unit **300** on, off or place it in sleep mode. Sleep mode will allow the sensor unit **300** to conserve its power by gathering information at a slower rate, only gathering preselected information, turning off the display while continuing to monitor parameters and collect data, or any combination thereof. Buttons **304a**, **304b**, and **304c** are coupled to the display system **400**. A clinician can select the desired option in the display system **400** by scrolling through the available options with buttons **304a** and **304b**, and then selecting the desired option or options with button **304c**. In another embodiment, the display screen **402** may be a touch screen, which will allow the clinician to select the desired option or options by touching display screen **402**. Also, in that embodiment sensor **300** can include a retractable touch pen (not shown) that would be housed in sensor case **301**. The clinician can use the retractable touch pen to make the desired selection on the display screen **402** instead of using his/her finger. Additionally, sensor unit **300** may include light emitting diodes (LED's). The LED's can emit a combination of colors, such as red, yellow and green. The LED's will be coupled to display system so that the LED's will emit one of the available colors based off the status of the patient. The LEDs may indicate an alarm state for one or more particular parameters that is/are monitored by the sensors **260**, **262**, **264**, **266**, and **306**. The alarm state is user programmable.

Turning to FIG. **9**, display system **400** includes a data processor **401** and display screen **402**. To receive various information and transform the received information to generate an output, display system **400** may utilize one or more of the controllers described above in reference to display system **200** (FIG. **6**). The data processor **401** may include a central processing unit, user interface, memory, sensor, and wireless component. The data processor **401** functions the same way as data processor **201**, as described above. Additionally, the user interface included in data processor **402** is the same or similar to the user interface included in the data processor **201** described above. The display screen **402** (FIG. 7) may include a liquid crystal distal, a light emitting diode display or the like. Also, the display screen **402** may be a touch screen.

A network interface car (NIC) or other suitable network interface utilizes any known communication methods for transmitting and/or receiving data to or from sensor **306**.

Any of the herein described methods, programs, algorithms or codes may be converted to, or expressed in, a programming language or computer program. A "Programming Language" and "Computer Program" is any language used to specify instruction to a computer, and includes (but is not limited to) these languages and their derivatives: Assembler, Basic, Batch files, BCPL, C, C+, C++, Delphi, Fortran, Java, JavaScript, Machine code, operating system command language, Pascal, Perl, PL1, scripting languages, Visual Basic, meta-languages which themselves specify programs, and all first, second, third, fourth, and fifth generation computer languages. Also included are database and other data schemas, and any other meta-languages. For the purpose of this definition, no distinction is made between languages which are interpreted, compiled, or use both compiled and interpreted approaches. For the purpose of this definition, no distinction is made between compiled and source versions of a program. Thus, reference to a program, where the programming language could exist in more than one state (such as source, compiled, object, or linked) is a reference to any and all such states. The definition also encompasses the actual instructions and the intent of those instructions.

Any of the herein described methods, programs, algorithms or codes may be contained on one or more machine-readable media or memory. The term "memory" may include a mechanism that provides (e.g., stores and/or transmits) information in a form readable by a machine such a processor, computer, or a digital processing devices. For example, a memory may include a read only memory (ROM), random access memory (RAM), magnetic disk storage media, optical storage media, flash memory devices, or any other volatile or non-volatile memory storage device. Code or instructions contained thereon can be represented by carrier wave signals, infrared signals, digital signals, and by other like signals.

Sensor **306** may gather data about the fluids (e.g., liquid or gas) passing through the chest tube **108**. Sensor **306** may be a pressure senor for monitoring the fluid pressure within the chest tube **108**. Pressure sensor **306** will function the same or similar to the pressure sensor **260** as described above. Additionally, sensor **306** may be capable to monitor the fluid flow rate. Flow rate sensor **306** will function the same or similar to the flow rate sensor **260** as described above. Sensor **306** may be capable of monitor other characteristics of the fluids passing through the chest tube **108**. Sensor **306** may be able to differentiate the types of fluids exiting from the patient's chest cavity. Also, sensor **306** may be able to identify the presence and volume or concentration of blood, if any, in the contents of the chest tube **108**. When sensor **306** is gathering information regarding the presence of blood, sensor **306** will function the same or similar to the sensor **266**. Sensor **306** may have the capabilities of measuring the pH level of the fluids, as well as the ability to detect the presence of CO₂. When sensor **306** is gathering information regarding the pH level and/or CO₂ level, sensor **306** will function the same or similar to senor **262** and/or sensor **264**. Also, if blood is present in the fluid within the chest tube **108**, sensor **306** may have the ability to detect the glucose levels. Additionally, sensor **306** may have the capability of identifying the color and turbidity of the fluids within the chest tube **108**. In gathering information regarding the color and turbidity of the fluids, senor **306** will function the same or similar to sensor **266**. Measuring the pH and glucose levels and detecting the color and turbidity of the fluids within the chest tube **108** may enable the clinician to detect the presence of an infection. The detection of carbon dioxide may indicate an air leak in the patient's lung. Sensor unit **300** communicates with sensor **306** and display unit **400** to analyze the data collected by sensor **306** and uses algorithms to determine if any of the collected data were outside of the normal range and if so, sends a communication to the appropriate clinician.

Sensor unit **300** may communicate with the motorized chest drainage system **100** via any conventional wireless technology. Also, the sensor unit **300** may communicate with the motorized chest drainage system **100** through a wired port on the sensor unit **300**, such as a USB port or any similar technology. The sensor unit **300** may also communicate with a mobile device, such as a mobile phone or pager. This communication may be accomplished by using any conventional wireless technology. The clinician may preselect the information the sensor unit **300** will communicate with the motorized chest drainage system **100** and/or the mobile device.

The sensor unit **300** can work in tandem with motorized chest drainage system **100** or can be placed on a non-smart chest drainage system. Also, the motorized chest drainage system **100** can fully function without the inclusion of sensor unit **300**. When sensor **300** is included in the motorized chest drainage system **100**, sensor **300** may augment the function of the motorized chest drainage system **100**.

With reference to FIGS. **1-6****,** use of the motorized chest drainage system **100** is discussed. During the course of a thoracic surgical procedure or in a situation where fluids (e.g., liquid or gas) need to be removed from a patient's thoracic cavity, a clinician inserts the articulating tip **111** of the motorized chest drainage system **100** through an opening in tissue (e.g., an incision) into the pleural cavity. The articulating tip **111** may be inserted anteriorly, posteriorly, or laterally into the pleural cavity. Once the articulating tip **111** is placed within the pleural cavity, the clinician may adjust the placement of the articulating tip **111** by remotely controlling the actuation assemblies **130**, selecting a predetermined oscillation pattern of the articulating tip, or selecting a user defined pattern of tip movement. Prior to use, the practitioner may program a user defined pattern of tip movement in view of the procedure to be performed. Initially, the practitioner may manually control the articulating tip **111** and the removal of fluids until the patient's condition is such that automatic control of the articulating tip **111** is possible or desirable. During use, the motorized chest drainage system **100** may be temporarily suspended in order to drain collected fluids from fluid reservoir **109**. Alternatively, the fluid reservoir **109** may be coupled to a drainage system allowing for continued and uninterrupted operation.

In any of the embodiments disclosed herein, the motorized chest drainage system **100** can be programmed to have an autonomous, randomly oscillating tip with intelligence. The movement of the articulable tip **111** can randomly oscillate, or follow a simple algorithm that defines a pattern, such as circular, criss-crossing movement, up and down or side to side reciprocation, or a combination of these. Thus, the motorized chest drainage system **100** can have a flexible or articulated tube that is motorized and has preprogrammed movements. Furthermore, in the presently disclosed embodiments, the sensor positioned on the suctioning tip may be a pressure sensor, a flow rate sensor, a pH sensor, a gas sensor, or a fluid content sensor. The pressure sensor may be used to determine the pressure of the fluids in the pleural space and can detect leaks in the lung.

A gas sensor can be used to determine if there is a leak by detecting the presence or quantity of CO₂, O₂, or other gases. A fluid content sensor can detect the presence of blood or another fluid, and the pH sensor can be used to identify a possible infection.

Pressure changes at the articulable tip **111** can also indicate the presence of a blockage, or that the articulable tip **111** has suctioned tissue or other particulate matter in the pleural space. In the event that the pressure sensor indicates a blockage or that the articulable tip **111** is stuck on tissue, the motorized chest drainage system **100** can be programmed to "puff" and blow air, CO₂ or another biocompatible gas or liquid through the chest tube **108** and then to move the articulable tip **111** in an opposite direction. In any of the embodiments disclosed herein, the motorized chest drainage system **100** can be programmed so that after detecting an undesirable reading from the sensors **260**, **262**, **264**, and **266**, the movement of the articulable tip **111** is changed, reversed in direction, or modified. In addition, a user of the motorized chest drainage system **100** can manually, or by interaction with an interface on the housing **140**, change the movement of the articulable tip **111**. In another example, the motorized chest drainage system **100** can be programmed so that the movement of the articulable tip **111** is automatically changed in response to an indication that movement is resisted. Such indication can come from movement and positioning sensors on the articulable tip **111**.

Smart programming, or artificial intelligence, can be included in the motorized chest drainage system **100**, in any of the embodiments. For example, the motorized chest drainage system **100** can track how often the articulable tip **111** becomes blocked or stuck, and establish a different pattern of movement on that basis. The motorized chest drainage system **100** can provide a report to the user, so that the user can understand that certain patterns of movement are undesirable and interact with the motorized chest drainage system **100** to make changes.

The entire contents of U.S. Patent Application Serial No. 62/275,829 filed January 7, 2016, is hereby incorporated by reference herein.

It is contemplated that the motorized chest drainage system **100** may use wireless communications to send configurable reports or notifications on the patient's condition to mobile communications devices, such as mobile phones or pagers, as carried by a clinician who may be monitoring the patient's condition. Such wireless notifications may alert a clinician that the chest tube **108** has become blocked, or disconnected from the patient, the presence of a possible infection, or a leak coming from the patient's lungs.

It will be understood that various modifications may be made to the embodiments disclosed herein. Therefore, the above description should not be construed as limiting, but merely as exemplifications of embodiments. Those skilled in the art will envision other modifications within the scope and spirit of the present disclosure.

The invention may be described by reference to the following numbered paragraphs:-
1. A chest drainage system, comprising:
   a flexible tube having proximal and distal ends;
   a tip positioned at the distal end of the flexible tube;
   a de-clogging mechanism operatively coupled to the flexible tube;
   an articulation assembly operatively coupled with the flexible tube, the articulation assembly is adapted to articulate the tip; and
   a control assembly which is operated by a motor, the control assembly operatively coupled with the articulation assembly.
2. The chest drainage system of Paragraph 1, further including a suction source including a sensor in operative communication with the suction source to collect data on suction pressure, fluid flow rate, or volume of fluid, or content type or any combination thereof.
3. The chest drainage system of Paragraph 2, further including a data processor to evaluate data collected by the sensor.
4. The chest drainage system of Paragraph 2, further including a fluid reservoir.
5. The chest drainage system of Paragraph 4, further having a case attached to the proximal end of the flexible tube, the case housing the suction source, the sensor, the data processor, the fluid reservoir and the control assembly.
6. The chest drainage system of Paragraph 5, wherein the case includes a display for displaying data collected by the data processor.
7. The chest drainage system of Paragraph 5, wherein the case also includes a power outlet or a battery, or both.
8. The chest drainage system of Paragraph 5, wherein the case also includes a control for the articulation assembly and a motor control.
9. The chest drainage system of Paragraph 1, further including a de-clogging mechanism.
10. The chest drainage system of Paragraph 1, wherein the articulation assembly is remotely operated.
11. The chest drainage system of Paragraph 1, wherein the articulation assembly is programmed to have a set oscillation pattern.
12. The chest drainage system of Paragraph 1, wherein the articulation assembly is programmed to have a user defined pattern.
13. The chest drainage system of Paragraph 1, further including a detachable sensor unit disposed in-line with the flexible tube.
14. The chest drainage system of Paragraph 13, wherein the detachable sensor unit includes a sensor to gather data about the pressure, flow rate, pH, presence of blood, carbon dioxide levels and glucose levels, or any combination thereof.
15. The chest drainage system of Paragraph 14, wherein the detachable sensor unit includes a display system, the display system includes a data processor to evaluate the date collected by the sensor and a display unit to display data collected by the sensor.
16. The chest drainage system of Paragraph 15, wherein the detachable sensor unit wirelessly communicates the data collected by the sensor to the chest drainage system, a mobile device, or any combination thereof.
17. The chest drainage system of Paragraph 13, wherein the detachable sensor unit includes a battery which provides power to the detachable sensor unit.
18. 27. A method for placing a chest drainage system, comprising:
   a flexible tube having proximal and distal ends;
   a tip positioned at the distal end of the tube;
   an articulation assembly operatively coupled with the flexible tube, the articulation assembly is adapted to articulate the tip;
   a control assembly which is operated by a motor, the control assembly operatively coupled with the articulation assembly; and
   a suction source including a sensor in operative communication with the suction source to collect data on suction pressure, fluid flow rate, or content type or any combination thereof;
   inserting the tip within a pleural cavity;
   operating the articulation assembly to adjust the tip within the pleural cavity to secure a desired anatomical location; and
   adjusting the tip within the pleural cavity after a pleural effusion is detected.

## Claims

1. A chest drainage system, comprising:
a flexible tube having proximal and distal ends;
a tip positioned at the distal end of the flexible tube;
a de-clogging mechanism operatively coupled to the flexible tube;
an articulation assembly operatively coupled with the flexible tube, the articulation assembly is adapted to articulate the tip; and
a control assembly which is operated by a motor, the control assembly operatively coupled with the articulation assembly.

2. The chest drainage system of Claim 1, further including a suction source including a sensor in operative communication with the suction source to collect data on suction pressure, fluid flow rate, or volume of fluid, or content type or any combination thereof.

3. The chest drainage system of Claim 2, further including a data processor to evaluate data collected by the sensor.

4. The chest drainage system of Claim 2 or claim 3, further including a fluid reservoir.

5. The chest drainage system of Claim 4, further having a case attached to the proximal end of the flexible tube, the case housing the suction source, the sensor, the data processor, the fluid reservoir and the control assembly.

6. The chest drainage system of Claim 5, wherein the case includes a display for displaying data collected by the data processor.

7. The chest drainage system of Claim 5 or claim 6, wherein the case also includes a power outlet or a battery, or both.

8. The chest drainage system of any of claims 5 to 7, wherein the case also includes a control for the articulation assembly and a motor control.

9. The chest drainage system of any preceding claim, further including a de-clogging mechanism.

10. The chest drainage system of any preceding claim, wherein the articulation assembly is remotely operated.

11. The chest drainage system of any preceding claim, wherein the articulation assembly is programmed to have a set oscillation pattern, or wherein the articulation assembly is programmed to have a user defined pattern.

12. The chest drainage system of any preceding claim, further including a detachable sensor unit disposed in-line with the flexible tube.

13. The chest drainage system of Claim 12, wherein the detachable sensor unit includes a sensor to gather data about the pressure, flow rate, pH, presence of blood, carbon dioxide levels and glucose levels, or any combination thereof; and/or wherein the detachable sensor unit includes a display system, the display system includes a data processor to evaluate the date collected by the sensor and a display unit to display data collected by the sensor.

14. The chest drainage system of Claim 13, wherein the detachable sensor unit wirelessly communicates the data collected by the sensor to the chest drainage system, a mobile device, or any combination thereof.

15. The chest drainage system of Claim 14, wherein the detachable sensor unit includes a battery which provides power to the detachable sensor unit.
